# EUROPÄISCHE PATENTSCHRIFT

(11) **EP 3 957 964 B1**
(45) Veröffentlichungstag und Bekanntmachung des Hinweises auf die Patenterteilung: **03.07.2024**
(21) Anmeldenummer: 21189651.9
(22) Anmeldetag: 04.08.2021
(51) Int. Cl.: G01J 5/02, A61D 13/00, A61B 5/00

(54) **MOBILE VORRICHTUNG UND VERFAHREN ZUR BERÜHRUNGSLOSEN ERMITTLUNG DER KÖRPERTEMPERATUR EINES TIERES**
MOBILE DEVICE AND METHOD FOR CONTACTLESS DETERMINATION OF THE BODY TEMPERATURE OF AN ANIMAL
DISPOSITIF MOBILE ET PROCÉDÉ DE DÉTERMINATION SANS CONTACT DE LA TEMPÉRATURE CORPORELLE D'UN ANIMAL

(30) Priorität: 18.08.2020 DE 102020121649
(43) Veröffentlichungstag der Anmeldung: 23.02.2022
(73) Patentinhaber: Polten, Bernhard, Alfter (DE)
(72) Erfinder: Polten, Bernhard, Alfter (DE)
(74) Vertreter: Weickmann & Weickmann PartmbB

(56) Entgegenhaltungen:
- WO-A1-2019/166986
- DE-A1-102016 222 079
- US-A- 5 285 396
- US-A1- 2017 202 185
- US-A1- 2018 094 983
- US-A1- 2019 307 106

## Beschreibung

### I. Anwendungsgebiet

Die Erfindung betrifft eine Vorrichtung und ein Verfahren zur Ermittlung der Körpertemperatur eines Tieres z.B. Nutztieres oder Wildtieres, im Laufstall, auf der Weide, im Gehege oder im Freiland.

### II. Technischer Hintergrund

Bei der Beurteilung des Wohlbefindens und des Gesundheitszustandes eines Tieres ist neben allgemeinen Symptomen einer der aussagekräftigsten Parameter die Körpertemperatur der Tiere.

Insbesondere bei größeren Herden von Tieren ist die kontinuierliche Überwachung des Gesundheitszustandes der einzelnen Tiere eine Aufgabe, die große Sachkunde erfordert und mit erheblichem Arbeitsaufwand verbunden ist. Trotzdem ist es sowohl im Sinne einer artgerechten Tierhaltung (Tierschutz) als auch aus ökonomischen Erwägungen unbedingt notwendig, gesundheitliche Probleme frühzeitig zu erkennen, um frühzeitig Gegenmaßnahmen ergreifen zu können und um dem Tier Leid zu ersparen (Früherkennung) und das Risiko eines Übergreifens auf andere Tiere (z.B. Tierseuchenbekämpfung) sowie den Behandlungsbedarf zu minimieren.

Als Standardmethode, um die Körpertemperatur zu bestimmen, wird in der Praxis gewöhnlich die manuelle rektale Messung angewandt. Sie ist - besonders bei größeren Herden - mit einigen grundsätzlichen Problemen und viel Aufwand an Fachpersonal verbunden.

Eine weitere in der Praxis angewandte Möglichkeit besteht - nur bei bestimmten Tierarten, insbesondere Wiederkäuern - darin, einen sog. Temperaturbolus in den Magen des Tieres einzusetzen, den beispielsweise eine Kuh dauerhaft im Pansen trägt und der die Temperatur über Funk an eine stationäre Empfangsstation überträgt.

Der Bolus muss veterinärmedizinisch implantiert werden; ist nicht wiederverwendbar und die Temperaturumgebung des Bolus im Magen schwankt abhängig vom Verdauungszustand erheblich.

Eine weitere Möglichkeit zur Überwachung besteht darin, einen Temperatursensor zur Messung der Oberflächen-Temperatur an einer Stelle des Körpers des Tieres zu fixieren oder zu implantieren.

Jedoch ist die Fixierung und/oder Implantierung selbst aufwändig, und bei einer Fixierung außen am Tier ist die Gefahr des Abreißens relativ groß und die Kapazität des Akkus oder der Batterie begrenzt.

Ferner könnte eine berührungslose Messung genutzt werden.

Diese konnten sich aber in der praktischen Anwendung bisher nicht durchsetzen, denn es fehlte an für Landwirte unkompliziert nutzbaren und technisch einfachen sowie vor allem zuverlässigen Lösungen.

Dies gilt für Tiere im Laufstall, im Gehege, auf der Weide oder erst recht für freilebende Wild-Tiere, die nicht ständig bestimmte Futterstellen oder Melkstände aufsuchen, denn berührungslose, automatisierte T-Messungen wären dort am einfachsten machbar, da hierbei das erste Problem, die Lokalisierung der Tiere, wegfällt und wegen der in etwa immer gleichen Position des Tieres in zum Beispiel einem Melkstand auch bei dort fest installiertem Temperatur-Sensor die Messstelle am Tier in etwa immer die gleiche wäre.

Die DE 10 2016 222 079 A1 beschreibt ein Stall-Reinigungsgerät für Großvieh, welches primär den Gang zwischen den Reihen von Liege-Boxen reinigt, und als Zusatzfunktion Sensoren aufweist, um sowohl den Stall-Zustand, insbesondere die Luft im Stall, zu überwachen als auch bei in Liegeboxen neben dem Gang liegenden Tieren deren Temperatur zu messen, um diese mit einem vorgegebenen Schwellwert zu vergleichen und bei Abweichung ein Alarmsignal abzugeben.

Ferner ist aus der US 2019/0307106 A1 ein Fahrzeug zum Einsatz in einem Hühnerstall bekannt, welches primär dafür vorgesehen ist, mittels eines Greifers tote oder kranke Hühner aus dem Stall zu entfernen sowie Proben von Futter und Wasser zu nehmen, als auch Eier einzusammeln. Zusätzlich wird damit mittels einer temperatur-empfindlichen Kamera die Körpertemperatur einzelner Tiere gemessen.

Ferner ist es aus der US 2017/0202185 A1 bekannt, mittels Flug-Drohnen und daran befestigter Kameras und Sensoren den Gesundheitszustand der Herde auf einer Farm, die sich im Freien bewegt, zu überwachen.

### III. Darstellung der Erfindung

### a) Technische Aufgabe

Es ist daher die Aufgabe gemäß der Erfindung, eine Vorrichtung sowie ein Verfahren zum einfachen und kostengünstigen, berührungslosen Messen der Oberflächen-Temperatur an einem frei laufenden Tier zur Verfügung zu stellen, das auch für Routinemessungen und regelmäßige Messungen geeignet ist.

### b) Lösung der Aufgabe

Diese Aufgabe wird durch die Merkmale der Ansprüche 1 und 14 gelöst. Vorteilhafte Ausführungsformen ergeben sich aus den Unteransprüchen.

Eine erfindungsgemäße Vorrichtung zur berührungslosen Ermittlung der Körpertemperatur eines freilaufenden Tieres wird im Anspruch 1 definiert.

Das Sensor-Fahrzeug kann ein, vorzugsweise selbst angetriebenes, Landfahrzeug sein, insbesondere ein Rad-Fahrzeug, ein Raupen-Fahrzeug oder ein Schreit-Fahrzeug, oder auch ein Luftfahrzeug, insbesondere eines, das in der Luft stehen kann, etwa ein Hubschrauber, ein Multikopter mit mehreren in horizontalen Ebenen rotierenden Drehflügeln, oder ein Ballon.

Gerade bei Sensor-Fahrzeugen in Form von Land-Fahrzeugen kann es sinnvoll sein - gerade bei der Versorgung von Nutztieren - das Sensorfahrzeug funktionsvereinigt mit einem anderen in der Umgebung der Tiere zu einem anderen Zweck ohnehin herumfahrenden Service-Fahrzeug auszubilden.

Solche, insbesondere frei fahrenden, gegebenenfalls sogar autonom gesteuerten, angetriebenen Service-Fahrzeuge können selbstfahrende Reinigungs-Roboter oder Fütter-Roboter sein, die die Tiere versorgen.

Da diese bereits über ein Fahrwerk mit Antrieb und Lenkung und gegebenenfalls eine Steuereinheit dafür verfügen, müssen zusätzlich lediglich die übrigen erforderlichen Bestandteile des Sensor-Fahrzeuges wie insbesondere Sensoren und/oder Kameras und/oder Tier-Identifikationsmittel und/oder Auswerteeinheit daran oder darin zusätzlich verbaut werden.

Die Vorrichtung umfasst ferner eine Lokalisierungs-Einheit, um zu vermessende Tiere auch in einem weitläufigen Areal zunächst ausfindig zu machen.

Die Lokalisierung-Einheit kann ebenfalls an dem Sensor-Fahrzeug angeordnet sein, oder an einem separaten Lokalisierungs-Fahrzeug befestigt sein, welches insbesondere keinen Temperatur-Sensor aufweist und immer in großem Abstand, insbesondere in großer Höhe über den Tieren verbleibt, beispielsweise ein in der Luft stehender Multikopter, oder über ein Seil am Boden befestigter, Ballon.

Dieser kann beispielsweise über seine Wärmebild-Kamera grob die Position der einzelnen Tiere bestimmen und an das Sensor-Fahrzeug weitergeben, das die einzelnen Tiere aufsucht und die Temperatur-Messung vornimmt.

Eine Lokalisierung ist eventuell auch gar nicht notwendig, wenn die Tiere mit einem Lokalisierungs-Sender ausgestattet sind, so dass eine Zentraleinheit und/oder das Sensor-Fahrzeug, die mit diesem Lokalisierungs-Sender Verbindung aufnehmen kann, jederzeit die Position des Tieres kennt oder ermitteln kann.

Als Temperatursensoren kommen alle gebräuchlichen Temperatursensoren in Frage, die eine Oberflächen-Temperatur berührungslos bestimmen können, vorzugsweise wird jedoch ein Pyrometer verwendet, insbesondere ein Infrarot-Pyrometer, und/oder eine Wärmebildkamera und/oder ein Infrarotsensor.

Die Auswerteeinheit kann eine entsprechend programmtechnisch eingerichtete Datenverarbeitungseinheit sein.

Da die Auswerteeinheit Temperaturdaten bezogen auf ein bestimmtes Tier speichert, sind der Auswerteeinheit auch alle älteren Temperaturdaten von jedem einzelnen Tier bekannt und insbesondere bevorzugt in Verbindung mit anderen Daten, wie beispielsweise Zeitpunkt der Messung, verfügbar. Dadurch lassen sich leicht und automatisiert, Tiere mit seitherigen Temperaturveränderungen ermitteln, häufig noch bevor weitere Krankheitssymptome auftreten und / oder andere Tiere angesteckt werden.

Eine Anzeigeeinheit dient dem Zweck, dass der Benutzer über die Temperaturwerte in Kenntnis gesetzt wird und ggfs. erforderliche Schritte einleiten kann.

Zu diesem Zweck kann die Sende- und/oder Anzeigeeinheit eine tatsächliche physische Anzeigeeinheit sein, die optisch anzeigt, beispielsweise durch Anzeigen der Zahlenwerte oder über Symbolik, welche Temperatur gemessen wurde und/oder ob diese problematisch ist.

Da Pyrometer wenig Mess-Zeit benötigen, sind sie beispielsweise auch dann zu einer Messung in der Lage, wenn das Sensor-Fahrzeug lediglich am Tier vorbeifährt oder vorbeifliegt.

Das Sensor-Fahrzeug umfasst mehrere, einzeln hinsichtlich ihrer Messrichtung ansteuerbare, Temperatur-Sensoren, um beispielsweise bei in Gruppen zusammenstehenden Tieren an mehreren Tieren gleichzeitig eine Messung durchführen zu können.

Der Temperatursensor umfasst mindestens zwei Sensorelemente, die die Oberflächen-Temperatur, insbesondere gleichzeitig, an unterschiedlichen Stellen des Körpers des Tieres und/oder aus unterschiedlichen Richtungen bezogen auf den Körper des Tieres messen. Damit kann mit mindestens einem Sensorelement an einer Stelle des Körpers des Tieres gemessen werden, auch wenn die für mindestens ein anderes Sensorelement vorgesehene Messstelle verschmutzt oder nicht sichtbar, z.B. durch den Körper des Tieres selbst oder eines anderen Tieres oder Gegenstandes verdeckt ist.

Ferner kann durch das Vorsehen von mindestens zwei Sensorelementen und einem Abgleich von deren Messergebnissen auch leichter eine Fehlfunktion eines der Sensorelemente erkannt werden.

Bevorzugt ist dementsprechend die Auswerteeinheit, insbesondere deren Software, eingerichtet, problematische Diskrepanzen zwischen den Messungen der mindestens zwei Sensorelemente zu ermitteln und entsprechende Hinweis- und/oder Warnsignale zu erzeugen, zu senden und ggfs. anzuzeigen, beispielsweise mittels der Anzeigeeinheit.

Bevorzugt können bei der Nutzung mehrerer Sensorelemente diese mit erheblich verschiedenen Messrichtungen eingerichtet werden.

Die Vorrichtung umfasst ferner ein Tieridentifikationsmittel, welches bevorzugt einen Scanner, insbesondere einen Barcode-Scanner, einen RFID-Scanner, einen Ohrmarken-Scanner und/oder eine Kamera umfasst, wobei das Tieridentifikationsmittel auch eine, insbesondere software-gestützte, insbesondere eine künstliche Intelligenz (KI) nutzende, Identifikationseinheit, bevorzugt eingerichtet zur Gesichtserkennung oder Körpererkennung, etwa an Hand von Umrissen oder natürlichen Kennzeichnungen wie Fellfarbflecken, umfasst.

Alternativ oder zusätzlich kann die erfindungsgemäße Vorrichtung auch Mittel zur Eingabe und/oder Auswahl einer Tieridentität durch den Nutzer umfassen.

In der Tierhaltung werden Tiere üblicherweise mit entsprechenden Identifikationselementen wie Ohrmarken versehen, so dass die Zuordnung der Temperaturmessung zu einem bestimmten Tier am einfachsten mittels Identifikation des ohnehin vorhandenen Identifikationselements geschieht.

Grundsätzlich kann bei Gesichtserkennungssoftware erfindungsgemäß eine Kamera als Tieridentifikationsmittel dienen. Es ist erfindungsgemäß auch denkbar, dieselbe Kamera als Tieridentifikationsmittel und als Temperatursensor zu nutzen, beispielsweise indem die Identifikation anhand des Infrarotbildes erfolgt oder durch Nutzung einer sowohl infrarotes als auch sichtbares Licht aufzeichnenden Kamera.

Die Anzeigeeinheit, die in aller Regel entfernt vom Sensor-Fahrzeug in einer Leit-Zentrale angeordnet ist, kann zur physikalischen Reaktion z. B. optischen Anzeige beispielsweise Leuchtmittel in Form einer Ampel mit grünen, gelben und roten Leuchtmitteln umfassen und/oder einen Lautsprecher zur Abgabe eines akustischen Warnsignals, falls eine über einem vorgegebenen Schwellwert vorliegende Temperatur oder Diskrepanz zu einer früher gemessenen Temperatur festgestellt wird.

Eine Messung in einem Normbereich führt zu einem grünen Licht, wodurch ein Nutzer sieht, dass bei dem gerade vermessenen Tier die Temperatur in einem Normalbereich liegt oder in Abhängigkeit von vorherigen Messwerten unauffällig ist, z. B. bei einem Tier mit gerade abklingender Krankheit am Absinken ist, sodass keine weiteren Maßnahmen erforderlich sind.

Ein gelbes Licht wird beispielsweise erzeugt, wenn die Messwerte es sinnvoll erscheinen lassen, dass ein Tier noch einmal optisch durch den Nutzer/Bediener der Anlage geprüft werden sollte.

Ein rotes Licht wird beispielsweise erzeugt, wenn die Temperatur des Tieres so ist, dass unmittelbar Maßnahmen erfolgen müssen, wie beispielsweise eine Kontrolle und/oder Separierung des Tieres und/oder die Hinzuziehung eines Veterinärmediziners.

Alternativ kann aber auch vorgesehen sein, dass nur bei entsprechender Auffälligkeit der Messung ein Signal erzeugt wird.

Ebenfalls kann bei der vorher geschilderten Nutzung von mehreren Sensorelementen als Temperatursensor vorgesehen sein, mehrere separate Signalmittel vorzusehen, die jeweils einzelnen oder bestimmten Gruppen von Sensorelementen zugeordnet sind. Damit kann beispielsweise jeweils das Messergebnis von beiden Seiten des Tieres her angezeigt werden.

In einer bevorzugten Variante der integralen Ausbildung ist der Temperatursensor als Aufsteckeinheit oder Teil einer Aufsteckeinheit ausgebildet, insbesondere zum Aufstecken und Verbinden mit einem Smartphone, wobei das Smartphone besonders bevorzugt eingerichtet ist um als Auswerteeinheit und/oder Anzeigeeinheit zu dienen, wobei bevorzugt die Stromversorgung des Temperatur-sensors über die Verbindung mit dem Smartphone und/oder mittels Batterie/Akku erfolgt.

Da Smartphones ohnehin weitverbreitet sind, kann damit eine erfindungsgemäße Vorrichtung mit minimalen Hardwareanpassungen hergestellt werden.

Ein solches Smartphone mit einer solchen Aufsteck-Einheit kann am oder im Sensor-Fahrzeug befestigt werden.

Zusätzlich kann ein Nutzer bei einer ohnehin regelmäßig stattfindenden optischen Inspizierung/Durchzählung der Tiere sein zuvor per App eingerichtetes Smartphone mit einer entsprechenden Aufsteckeinheit versehen und jedes Tier nacheinander damit vermessen, wobei das Ausrichten des Temperatur-Sensors auf eine geeignete Stelle am Körper des Tieres dabei vorzugsweise nicht automatisch, sondern manuell durch den entsprechend fachkundigen Nutzer erfolgt.

Bevorzugt kann die Aufsteckeinheit auch weitere und/oder alle der zuvor oder nachfolgend genannten Komponenten der erfindungsgemäßen Vorrichtung umfassen, beispielsweise eine Aufsteckeinheit mit einer skalierten aus LEDs gebildeten Ampel als Teil der Anzeigeeinheit.

Bevorzugt ist die Auswerteeinheit dazu eingerichtet, basierend auf Oberflächen-Temperaturen eines bestimmten Tieres Folgemaßnahmen auszulösen, wie beispielsweise Änderung/Markierung eines Datenbankeintrags des bestimmten Tieres und/oder Benachrichtigungen, insbesondere elektronische, und/oder Verabreichung von besonderen Tierpflegemitteln oder Medikamenten.

Dadurch lassen sich bei einigen Tierarten übliche Standardmaßnahmen bei Auffälligkeiten automatisiert realisieren.

Hierbei kann die Auswerteeinheit insbesondere auch eingerichtet sein, die Folgemaßnahmen in Abhängigkeit von anderen Daten, insbesondere früheren Messwerten, auszulösen.

Bevorzugt umfasst die erfindungsgemäße Vorrichtung oder die gekoppelte, zuständige Zentralstation ferner eine Datenverarbeitungseinheit zur Speicherung und Pflege eines Tierdatenbestandes, der insbesondere aus den gemessenen Temperaturen besteht.

Insbesondere können für alle genannten Aufgaben/Vorgänge je nach konkreter Realisierung ganz oder teilweise oder auch gleichzeitig Zentralstation und/oder Auswerteinheit entsprechend eingerichtet sein, ggfs. auch Informationen an andere Betriebsangehörige, betreuende Firmen oder Betreuungsexperten weitergegeben werden.

Beispielsweise kommt es bei Nutzung von Aufsteckeinrichtungen auf Smartphones und mehreren Mitarbeitern in einer Anlage in Frage, dass die Aufsteckeinheit bzw. das Smartphone selbst als Auswerteeinheit hauptsächlich nur dahingehend eingerichtet sind, die gemessenen Temperaturdaten an eine Datenverarbeitungseinheit der Zentralstation weiterzuleiten, da eine Auswertung auf jeweils der Aufsteckeinheit bzw. dem Smartphone der einzelnen Mitarbeiter hinsichtlich einer Einheitlichkeit der Daten nicht unbedingt sinnvoll ist.

Es kann aber auch sinnvoll sein, um eine zu große Datenmenge an der Zentralstation zu vermeiden, einen erheblichen Teil der einfachen Funktionalitäten, wie beispielsweise Auslösung von Warnsignalen hinsichtlich einer akuten Gefährdung, durch eine entsprechende Einrichtung der Auswerteinheit zu realisieren, während die Zentralstation hauptsächlich der längerfristigen Datenpflege dient.

Bevorzugt ist die Auswerteeinheit dazu eingerichtet, in Abwesenheit eines Tieres Messungen von Neutraltemperaturen insbesondere an einer Testfläche auszuführen bzw. auszulösen, und bei ungewöhnlichen Neutraltemperaturen, ein Störungssignal auszulösen oder zu übertragen, insbesondere an die Anzeigeeinheit oder die Zentralstation.

Dabei richtet sich die gemessene Neutraltemperatur im Allgemeinen nach der Raumtemperatur bzw. der Raumtemperatur der vergangenen Stunden oder Minuten sowie insbesondere nach der Oberflächentemperatur der Testfläche, auf die der Temperatursensor ausgerichtet ist.

Damit wird durch solche Neutraltemperatur-Messungen der Temperatursensor regelmäßig überprüft und/oder kalibriert.

Dadurch können auch die Umgebungstemperatur und andere Umwelteinflüsse, z.B. durch Wind und Wetter, berücksichtigt werden.

Bevorzugt ist der Temperatursensor dazu eingerichtet, eine Entfernung zwischen Temperatursensor und der mindestens einen Mess-Stelle des Körpers des Tieres bzw. Tieres zu erfassen bzw. zu ermitteln, wobei bevorzugt die Auswerteeinheit und/oder die Datenverarbeitungseinheit ferner dafür ausgelegt ist, die Entfernung zu speichern und/oder eine korrigierte Temperatur unter Verwendung von wenigstens der gemessenen Oberflächen-Temperatur und der Entfernung zu bestimmen.

Alternativ oder zusätzlich können aber auch weitere Komponenten separat für die Entfernungsbestimmung vorgesehen sein, beispielsweise indem die erfindungsgemäße Vorrichtung auch einen Entfernungssensor umfasst.

Die Abstandsmessung kann dabei beispielsweise über Weg-Zeit-Messungen erfolgen, insbesondere auch direkt anhand von vom Temperatursensor ausgesandter Strahlung. Aber auch eine optische Bestimmung beispielsweise über ein von einer Kamera erfasstes Bild ist möglich.

Ferner können auch Drucksensoren oder Bewegungssensoren zur Bestimmung des Abstandes bzw. der Entfernung genutzt werden, wobei erfindungsgemäß, die Bestimmung nicht zwingend mit hoher Genauigkeit erfolgen muss.

Ebenfalls kann alternativ oder zusätzlich zu Entfernungsmessvorrichtungen vorgesehen sein, dass der Nutzer aufgefordert wird, die Entfernung abzuschätzen und einzugeben.

Die ermittelte Entfernung kann dabei in verschiedener Weise genutzt werden. Beispielsweise zur direkten Korrektur der gemessenen Oberflächen-Temperatur, oder als Daten hinsichtlich des Tieres, denn Bewegung und Körperhaltung des Tieres sind mit dem Gesundheitszustand korreliert.

Es können so auch systematische Fehler besser erkannt werden, beispielsweise, wenn bei größerer Entfernung im Mittel eine niedrigere oder höhere Temperatur gemessen wird als bei kleinerer Entfernung.

Ein erfindungsgemäßes Verfahren, welches bei Nutzung einer erfindungsgemäßen Vorrichtung in einer der geschilderten Varianten ausgeführt wird, wird in Anspruch 14 definiert.

Bevorzugt werden die Schritte in der im Anspruch 14 genannten Reihenfolge ausgeführt, wobei eine beliebige andere Reihenfolge und auch jeweils gleichzeitige Ausführung ebenfalls erfindungsgemäß ist, insbesondere eine andere Reihenfolge von Messen der Oberflächen-Temperatur eines Tieres und Identifizieren des Tieres und insbesondere auch gleichzeitiges Messen und Identifizieren.

Die Zuordnung der Daten zu einem Tier kann bei dem erfindungsgemäßen Verfahren beispielsweise durch manuelle Eingabe des Namens oder einer Nummer durch den Tierhalter oder Betreuer oder durch Lesen von Barcodes (z.B. auf der Ohrmarke) oder Scannen eines RFID Signales des Tieres (z. B. von Transponder) oder mittels Bilderkennung (Biometrie) erfolgen.

Beispielsweise werden bei der Überwachung von Wildtierbeständen ohnehin häufig quasi künstliche Futterstellen eingerichtet und per Kamera oder ähnlichem das Auftauchen eines Wildtieres an entsprechender Stelle registriert, um beispielsweise die Anzahl der Wildtiere zu überwachen.

Eine entsprechend eingerichtete künstliche oder auch natürliche Futter- oder Tränkstelle ist ebenfalls im Sinne der Erfindung als häufiger Aufenthaltsort von Tieren geeignet, der die Anwesenheit von Tieren an die Zentralstation meldet und kann entsprechend für eine einfache Lokalisierung der Tiere genutzt werden.

Zur Temperaturerfassung werden bevorzugt Infrarotmessungen bzw. -strahlung genutzt, denn die Körperinnentemperatur und die Körperoberflächentemperatur ist korreliert und zwar vom Grundsatz her im Sinne einer konstanten Temperaturdifferenz, wobei es Abweichungen geben kann (beispielsweise variierende Felldicke, Verschmutzungen der Körperoberfläche, etc.). Abweichungen lassen sich jedoch durch geeignete Algorithmen erkennen und eliminieren, wofür die Auswerteeinheit und/oder die Zentralstation entsprechend eingerichtet sein können.

Mit einer Infrarotkamera als Temperatursensor kann flächenhaft die Temperatur ermittelt und ein sogenanntes Wärmebild erzeugt werden. Damit können Temperaturen an vielen Temperaturmesspunkten bestimmt werden.

Mittels eines sogenannten Referenz-Strahles, erzeugt beispielsweise durch den Temperatursensor und/oder eine dazugehörige oder damit verbundene Referenz-Strahl-Komponente, kann die gemessene Temperatur rechnerisch korrigiert werden und so die Genauigkeit der Temperaturmessung gesteigert werden.

Der Temperatursensor ist bevorzugt so ausgebildet, dass er für die zu erwartenden Temperaturen (bei Säugetieren zwischen 25°C und 45°C) optimal geeignet ist.

Für die Fernkommunikation werden bevorzugt drahtlose Datenübertragungselemente vorgesehen bzw. verwendet, die z.B. über RFID oder Funk an einer Zentralstation bzw. Datenverarbeitungseinheit angemeldet werden können. Die hierfür benötigten RFID-Transponder sind in diesem Falle bevorzugte Bestandteile der erfindungsgemäßen Vorrichtung.

Die Zentralstation überwacht gegebenenfalls eine große Anzahl derartiger Sender. Diese sind mit einer geeigneten Energiequelle versehen.

### c) Ausführungsbeispiele

Ausführungsformen gemäß der Erfindung sind im Folgenden beispielhaft näher beschrieben. Es zeigen:
- **Figur 1a:**: ein Pferd mit verschiedenen Sensor-Fahrzeugen in der Nähe des Pferdes in der Seitenansicht,
- **Figur 1b:**: in der Aufsicht von oben einen Ballon 1 mit einer Reihe von Tieren, (hier Rindern oder Schafen, teilweise innerhalb und teilweise außerhalb dessen Detektions-Bereiches, sowie weiteren Sensor-Fahrzeugen,
- **Figur 2:**: ein Mobil-Telefon mit einer Aufsteck-Einheit,
- **Figur 3:**: eine nicht erfindungsgemäße Kombination aus einem Sensor-Fahrzeug und einem Service-Fahrzeug.

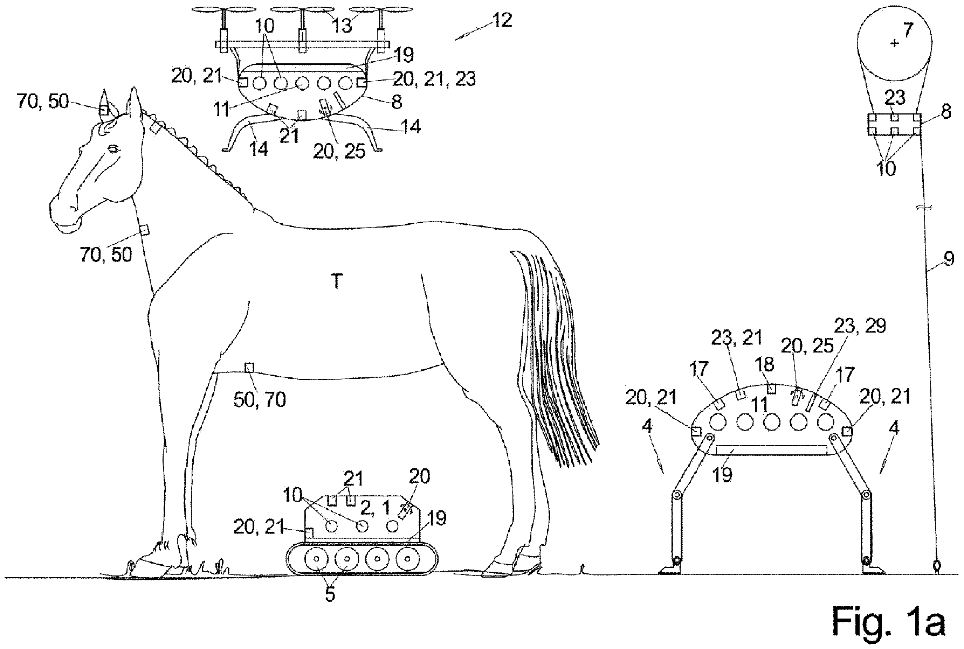

**Figur 1a** zeigt ein Pferd als Tier **T** zusammen mit drei möglichen Sensor-Fahrzeugen **1,** die einen Temperatur-Sensor **20** wie etwa einem Pyrometer tragen zur Bestimmung der Oberflächen-Temperatur des Tieres **T:**
Am Tier **T** sind am Ohr, an Ober- und Unterseite des Halses, im Nacken sowie an der Unterseite des Bauches mögliche Positionen für ein dort evtl. vorhandenes Tier-Identifikationsmittel **50** dargestellt, wie etwa ein eingepflanzter Chip oder eine Marke, insbesondere eine Ohrmarke.

Die dargestellten Positionen können auch Messstellen-Identifikationsmittel **70** sein, um potentielle Temperatur-Messstellen am Tier **T** zu markieren, beispielsweise ebenfalls in Form eines implantierten oder befestigten Chips, der vorzugsweise Signale ausgibt, die von einem entsprechenden Sensor des Sensor-Fahrzeuges **1** lokalisiert und angefahren werden kann.

Wenn es sich jedoch um Wildtiere handelt, kann es auch sein, dass diese keinerlei am Tier angeordnete Markierungen, weder für die Tier-Identifikation noch für die Bezeichnung einer möglichen Messstelle am Tier **T,** aufweisen.

Mögliche Sensor-Fahrzeuge **1** sind als lenkbare, vorzugsweise motorisch angetriebene, Land-Fahrzeuge, einerseits ein Raupen-Fahrzeug **2** - möglich wäre natürlich auch ein Fahrzeug mit Rädern - und andererseits ein Schreit-Fahrzeug **3** dargestellt.

Beim Raupen-Fahrzeug **2** trägt das Raupen-Fahrwerk - beidseits des Fahrzeuges sind mehrere in Fahrtrichtung hintereinander angeordnete Räder **5** vorhanden, über die eine Raupenkette **6** läuft - eine Gondel **8,** an und in der die wesentlichen Sensoren und weiteren Bauteile des Sensor-Fahrzeugs **1** untergebracht sind.

Beim Schreit-Fahrzeug **3** wird eine solche Gondel **8** von drei, vier oder mehr ansteuerbaren Beinen getragen, die jeweils aus mehreren Beinteilen, welche gelenkig miteinander verbunden sind, bestehen, wodurch eine Vorwärtsbewegung des Schreit-Fahrzeuges **3** durch Geh-Bewegungen ermöglicht wird.

Damit sollte klar sein, dass als Sensor-Fahrzeug **1** nicht nur solche Fahrzeuge gelten sollen, deren Vorwärts-Bewegung durch Fahren mittels Rädern bewirkt wird, sondern auch solchen mit anderen Formen von Vorwärts-Bewegungen, die aber lenkbar sein sollten.

Bei dem Luftfahrzeug in Form eines Multikopters **12** als Sensor-Fahrzeug **1** ist eine solche Gondel **8** - meist hängend - an einem Traggestell befestigt, welches die mindestens drei, in der Regel vier bis acht, in einer meist horizontalen Ebene rotierenden Rotoren **13** trägt, während sich am unteren Teil der Gondel **8** Standbeine **14** befinden, auf denen der Multikopter **12** nach dem Landen auf dem Untergrund steht.

Die Gondeln **8** der verschiedenen Sensor-Fahrzeuge enthalten neben den nicht dargestellten Antriebsmotoren für das jeweilige Fortbewegungsmittel - also Räder **5,** Raupen-Fahrwerk **5 + 6,** Schreit-Beine **4,** Rotoren **13** - die Energieversorgung hierfür, in der Regel einen elektrischen Akku **19,** da die Antriebsmotoren in der Regel Elektromotoren sind, sowie eine nicht dargestellte Steuerung, die sowohl diese Antriebsmittel steuert als auch die weiteren in der Gondel **8** enthaltenen Elemente, insbesondere Sensoren:
Zunächst mehrere Temperatur-Sensoren wie etwa Pyrometer **20,** deren Messrichtung von der Steuerung eingestellt wird.

Je nach Größenverhältnis zwischen dem Sensor-Fahrzeug **1** und dem zu messenden Tier **T** befinden sich die Temperatur-Sensoren **20** bei den Sensor-Land-fahrzeugen entweder am äußeren Umfang oder an der Oberseite der Gondel **8,** bei einem Luftfahrzeug wie einem Multikopter **12** dagegen primär an dessen Außenumfang und / oder Unterseite der Gondel **8.**

Der Temperatur-Sensor **20** kann auch zum Messen der Umgebungstemperatur benutzt werden, wenn nicht ist hierfür ein eigener Umgebungs-Temperatursensor an der Gondel **8** vorhanden.

Darüber hinaus tragen die Gondeln **8** vorzugsweise eine oder mehrere, vorzugsweise in ihrer Blickrichtung einstellbare, Kameras **10,** die zum Ermitteln der richtigen Annäherungsrichtung an das Tier **T** dient, insbesondere an eine geeignete Messstelle, und bei fehlendem Tier-Identifikationsmittel **50** auch zum Identifizieren des spezifischen Tieres anhand dessen Biometrie, insbesondere Statur, aber auch Umrisse, Proportionen oder andere Kennzeichen des Körpers, vorzugsweise jedoch Form und / oder Größe von dessen Kopf, insbesondere dessen Gesicht mittels einer speziellen Gesichts-Erkennungssoftware.

Darüber hinaus trägt die Gondel vorzugsweise mindestens einen Entfernungssensor **21,** um die Entfernung zum Tier **T,** insbesondere zur Temperatur-Messstelle, messen zu können, welche zur Beurteilung der gemessenen Temperatur wichtig ist.

Ferner trägt die Gondel **8** in aller Regel einen GPS-Sensor **23,** damit für die Zentralsteuerung, mit der das eine oder in der Regel die mehreren Sensor-Fahrzeuge **1** verbunden sind - die bei einem einzelnen Sensor-Fahrzeug **1** jedoch auch direkt in dem Sensor-Fahrzeug **1** untergebracht sein kann - jederzeit Standort und Ausrichtung des Sensor-Fahrzeuges **1** bekannt ist und über den EntfernungsSensor **21** auch des momentan zu vermessenden Tieres **T.**

Vorzugsweise kann die Gondel **8** auch einen Ortungssensor **29** aufweisen, der geeignet ist, auf ein Tier-Identifikationsmittel **50,** welches innerhalb seiner Reichweite liegt, zu reagieren und dessen Position zu ermitteln.

Die Gondel **8** kann ferner vorzugsweise eine Lichtstrahl-Quelle **25** tragen, die einen dünnen gerichteten Lichtstrahl **26** wie etwa einen Laserstrahl oder einen fokussierten Lichtstrahl **26** einer Leuchtdiode abgibt in Messrichtung des Temperatur-Sensors **20,** insbesondere des Pyrometers, sodass am Tier **T** durch diesen Lichtstrahl und dessen Auftreffpunkt die momentan vermessene oder zur Vermessung angepeilte Messstelle zu erkennen ist, was mit der parallel zur Messung durchgeführten Bild-Aufnahme durch die Kamera **10** nachträglich eine Verifizierung der Messstelle ermöglicht.

Einige dieser Sensoren und/oder Vorrichtungen können auch funktionsvereinigt ausgebildet sein, insbesondere indem ein Mehrfach-Sensor mehrere verschiedene Sensor-Aufgaben wahrnehmen kann.

Die Gondel **8** kann ferner einen Lautsprecher **11** aufweisen, der für das Tier vertraute und vertrauenswürdige Geräusche von sich gibt, um ein Weglaufen des Tieres **T** von dem Sensor-Fahrzeug **1** zu vermeiden, beispielsweise typische Laute eines Tier-Kindes dieser Tierart bei Annäherung an ein weibliches Tier oder Laute eines weiblichen Tieres dieser Gattung bei Annäherung an ein männliches Tier **T.**

Die Steuerung für den Fortbewegungs-Mechanismus des Sensor-Fahrzeuges **1** kann bei einem Land-Fahrzeug so ausgelegt sein, dass es ein Bewegungsmuster des Land-Fahrzeuges erzeugt, welches der zu vermessenden Tierart vertraut ist und für diese keine Gefahr darstellt, was vor allem bei Schreit-Fahrzeugen **3** möglich ist, insbesondere, wenn dessen Gondel **8** zusätzlich mit einem relativ dazu bewegbaren Kopfteil ausgestattet ist.

Die Land-Sensorfahrzeuge **2, 3** können auch optisch getarnt sein als z.B. Stein, Busch oder anderer für das Tier **T** bekannter und nicht als Gefahr empfundenen Gegenstand.

In diesem Zusammenhang kann die Gondel **8,** also das Sensor-Fahrzeug **1,** auch Düsen zum Abgeben eines Gases oder einer Flüssigkeit mit Geruchsstoffen enthalten, welche für das Tier **T** ebenfalls positiv besetzt sind.

Das Sensor-Fahrzeug **1** kann ferner eine Injektions-Vorrichtung **27** tragen, aus der ein Medikament dem Tier **T** injiziert werden kann, sei es mit Hilfe eines präparierten Pfeiles oder als Flüssigkeitsstrahl mit einer so hohen Geschwindigkeit und so geringem Querschnitt, dass die Flüssigkeit teilweise durch die Haut des Tieres **T** eindringt.

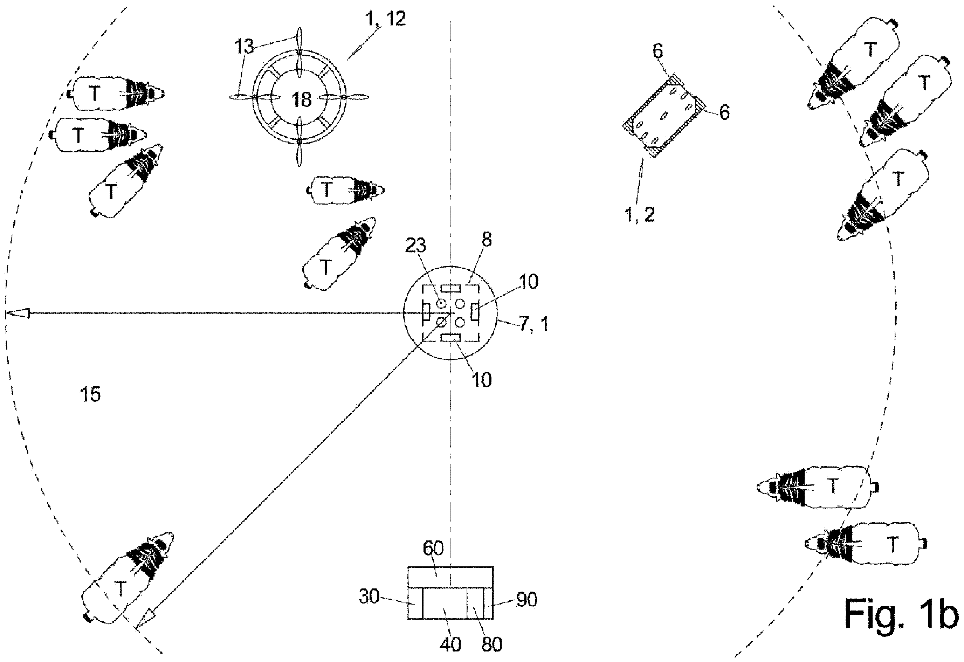

Weiterhin enthält das Sensor-Fahrzeug **1** vorzugsweise eine Sende-/EmpfangsEinheit **17,** mit der das Sensor-Fahrzeug **1** in Verbindung mit einer Zentral-Steuerung und / oder mit einem Ortungs-Fahrzeug für Tiere **T** steht, wobei in der Zentral-Steuerung auch die Auswerte-Einheit **30** und / oder die Anzeige-Einheit **40** und / oder die Eingabe-Einheit **41** für die Vorrichtung einschließlich aller vorhandener Sensor-Fahrzeuge **1** enthalten sein kann sowie eine entsprechende Speicher-Einheit, wie in **Figur 1b** dargestellt. Die Zentral-Einheit **60** kann und wird vorzugsweise mobil ausgebildet sein.

Das Sensor-Fahrzeug **1** kann ferner eine Markierungs-Vorrichtung **16** besitzen, um eine Markierung, beispielsweise eine Farbmarkierung, am Tier anzubringen, um es für einen Betreuer / Ranger zu kennzeichnen, der dann manuell beispielsweise Behandlungsmaßnahmen an dem Tier vornehmen kann.

Gerade landgebundene Sensor-Fahrzeuge **1** können auch eine Ausgabe-Vorrichtung für geringe Mengen eines von der Tierart besonders geschätzten Futters - sogenanntes Belohnungs-Futter - aufweisen und vor, während oder nach der Messung dem Tier **T** hinterlassen, um für die Zukunft die Scheu des Tieres **T** gegenüber dem Sensor-Fahrzeug **1** herabzusetzen und insbesondere Aggressionen des Tieres **T** gegenüber dem Sensor-Fahrzeug **1** herabzusetzen.

Wie zusammen mit der Aufsicht gemäß **Figur 1b** besser zu erkennen, kann vor dem Messen einzelner Tiere **T** durch Annähern des Sensor-Fahrzeuges **1** zunächst eine grobe Ortung der Tiere **T,** am besten aus der Luft, erfolgen, was entweder mittels eines Sensor-Fahrzeuges **1** in Form eines Luft-Fahrzeuges wie dem Multikopter **12** erfolgt, oder mit einem separaten Ortungsmittel wie beispielsweise einem schwebenden Ballon **7,** vorzugsweise in Form eines an einer Leine **9** gehaltenen, schwebenden Fessel-Ballons **7,** welcher ebenfalls eine Gondel **18** trägt, in der zumindest ein Ortungs-Sensor **29** - sei es in Form einer oder mehrerer Kameras **10** oder in Form eines Ortungs-Sensors **29,** der auf die an den Tieren vorhandenen Tier-Identifikationsmittel **50** anspricht - vorhanden ist.

Das Ortungs-Fahrzeug kann jedoch auch ein Klein-Flugzeug, insbesondere auch ein Modell-Flugzeug, sein, wobei die im Ortungs-Fahrzeug vorhandene Tier-Identifikationsmittel **50** jeweils einen bestimmten Detektions-Bereich **15** besitzen.

Sollen Flächen nach Tieren **T** abgesucht werden, die größere sind als der Detektionsbereich **15,** so muss das Ortungs-Fahrzeug entweder gesteuert beweglich sein relativ zum Untergrund, oder bei einem Ortungs-Fahrzeug wie einem Fesselballon muss dieser evtl. mehrfach an unterschiedlichen Positionen aufgelassen werden, vorzugsweise nachdem die in seinem bisher abgetasteten Detektionsbereich **15** ermittelten Tiere bereits von dem einen oder den mehreren Sensor-Fahrzeugen **1** vermessen wurden.

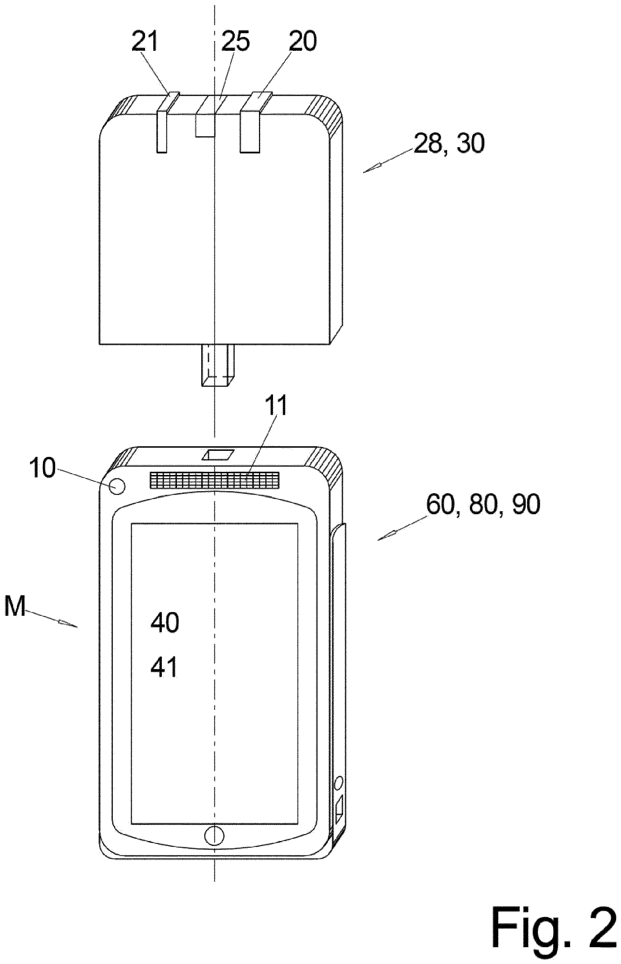

**Figur 2** zeigt ein übliches Mobil-Telefon **M** mit Kamera **10,** Lautsprecher **11,** einem Display **40,** welches gleichzeitig als Eingabe-Einheit **41** benutzt werden kann.

Ein solches Mobil-Telefon **M** kann entweder durch Aufspielen einer entsprechenden App, also eines Software-Programmes, oder durch zusätzliches Anschlie-βen, insbesondere Aufstecken, eines Aufsteck-Teils **28,** welches im Mobil-Telefon **M** nicht vorhandene Soft- und/oder Hardware enthält, entweder als Anzeige-Einheit **40** und / oder Eingabe-Einheit **41** für die Messvorrichtung verwendet werden:
Dann sieht der Bediener auf dem Display **40** des Mobil-Telefons **M** beispielsweise, was die oder eine bestimmte Kamera **10** des Sensor-Fahrzeuges 1 sieht, beispielsweise welches Tier **T** als Ziel ermittelt wurde, oder auf welche Körperstelle der Lichtstrahl **26** gerichtet ist, an dessen Auftreffstelle die Oberflächen-Temperatur des Tieres **T** gerade gemessen wird oder gemessen wurde oder um welches Tier **T** es sich handelt, indem die aus dem Tier-Identifikationsmittel **50** ausgelesenen Informationen oder das Ergebnis der Gesichtserkennungs-Software ebenfalls auf dem Display des Mobil-Telefons **M** angezeigt wird.

Auch eine manuelle Steuerung des Sensor-Fahrzeuges **1** insgesamt und / oder der Ausrichtung von dessen Temperatur-Sensor **20** und / oder der Kamera **10** und / oder der Injektions-Vorrichtung **27** und / oder der Markierungs-Vorrichtung **16** ist mittels des Mobil-Telefons **M** möglich.

Ein solches Mobil-Telefon **M,** insbesondere mit Aufsteckteil **28,** kann jedoch auch in definierter Position an oder in der Gondel **8** des Sensor-Fahrzeuges **1** angeordnet werden, um die im Mobil-Telefon **M** und dessen Aufsatzteil **28** vorhandenen Einrichtungen, insbesondere Sensoren, für das Sensor-Fahrzeug **1** zu nutzen, also
die eine oder die mehreren Kameras **10**
   und/oder
den Lautsprecher **11**
   und/oder
den GPS-Empfänger **23**
   und/oder
einen mit entsprechender App im Mobil-Telefon **M** vorhandenen Entfernungs-Sensor **21,**
   und / oder
eine im Aufsteckteil **28**vorhandene Lichtstrahl-Quelle 25
   und / oder
ein im Aufsteckteil **28**vorhandener Temperatur-Sensor **20,** insbesondere Pyrometer **20**
   und / oder
einen Temperatur-Sensor **20** für die Umgebungs-Temperatur
   und / oder
das Mikrofon des Mobil-Telefons **M,** um die von dem Tier T abgegebenen Töne aufzuzeichnen und / oder zu übermitteln.

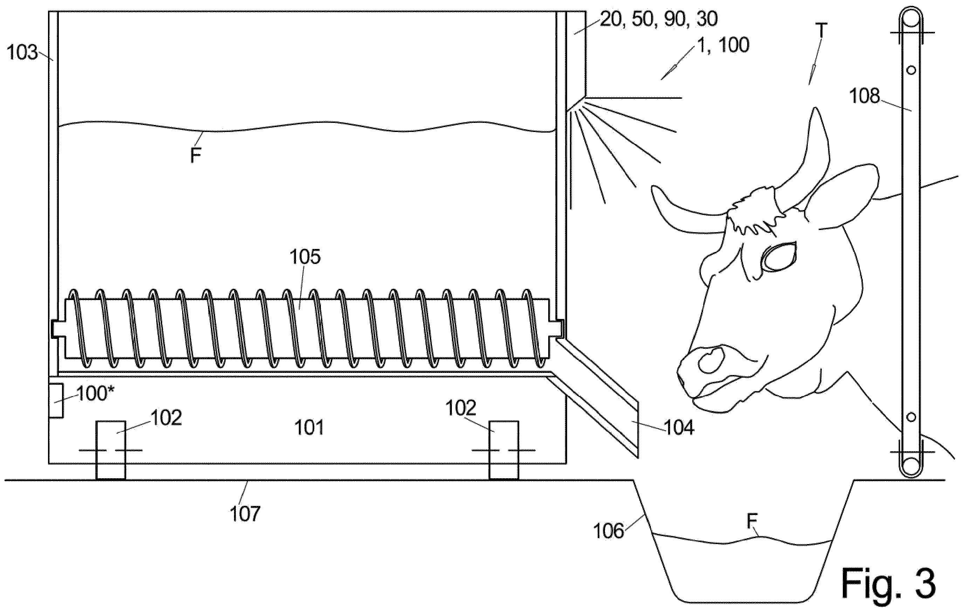

**Figur 3** zeigt, wie ein für die Nutztier-Betreuung ohnehin eingesetztes, Service-Fahrzeug 100, hier in Form eines selbstfahrenden, insbesondere autonom gesteuerten, Futter-Roboters 100, funktionsvereinigt ist durch Aufrüsten zu einem Sensor-Fahrzeug 1.

Der selbstfahrende Futter-Roboter 100, besitzt bereits einen Fahr-Antrieb 101 mit lenkbaren z.B. Rädern 102, einer Energieversorgung, meist in Form einer Batterie, sowie eine elektronische Steuerung 100* zum Steuern nicht nur des Fahr-Antriebes 101, sondern aller Funktionen des Futter-Roboters 100.

Denn dieser umfasst einen von oben nachfüllbaren Futter-Behälter 103, aus dem über eine seitliche Abgabeöffnung 104, beispielsweise mit Hilfe einer gesteuerten Fördererschnecke 105 im Inneren des Futter-Behälters 103, eine definierte Menge an Futter F ausgeworfen werden kann, beispielsweise in einen Futtertrog 106 unmittelbar vor dem Tier.

Die Futter-Menge - gegebenenfalls bei mehreren Futter-Bestandteilen in einzelnen Futter-Behältern auch die Zusammensetzung des Futters - kann auf das jeweilige Tier T abgestimmt sein.

Im dargestellten Anwendungsfall fährt der Futter-Roboter 100 entlang eines Futtertroges 106, beispielsweise auf dem Mittelgang 107 eines Stalles, wobei sich die einzelnen Tiere T entlang des Futtertroges 106 an durch ein Futter-Gitter 108 voneinander getrennten Fütter-Positionen befinden, an denen sie entweder angekettet sind - was keine Tier-Identifikation erfordert, da dann die Identität des Tieres an jeder Fütter-Position bekannt ist - oder ihre Fütter-Position frei wählen - was eine Tier-Identifikation erfordert.

Außer einem solchen Tier-Identifikationsmittel 50 umfasst der zum Sensor-Fahrzeug 1 aufgerüstete Futter-Roboter 100, wie das zuvor beschriebene Sensor-Fahrzeug 1, außer dem Tier-Identifikationsmittel 50
- wenigstens einen berührungslos arbeitenden Temperatursensor 20,
- insbesondere einen oder mehrere der weiteren zuvor beschriebenen Sensoren,
- insbesondere eine Auswerteeinheit 30 für die Temperatur-Messergebnisse,
- insbesondere eine Sende/Empfangseinheit 90 zum Übermitteln von Daten an eine Zentralstation oder eine Anzeigeeinheit.

Da das kombinierte Fahrzeug 1 + 100 ohnehin Futter F an die Tiere abgibt, kann dieses kombinierte Sensor-Fahrzeug 1 + 100 auch häufig benötigte, oral verabreichbare, Nahrungs-Ergänzungsmittel oder Medikamente an Bord haben, die bei Bedarf, insbesondere aufgrund des Temperatur-Messergebnisses, dem an das jeweilige Tier T abgegebene Futter F beigemischt werden kann.

### Bezugszeichenliste

- 1: Sensor-Fahrzeug
- 2: Radfahrzeug, Raupenfahrzeug
- 3: Schreitfahrzeug
- 4: Bein
- 5: Rad
- 6: Raupenkette
- 7: Ballon, Fesselballon
- 8: Gondel
- 9: Leine
- 10: Kamera
- 11: Lautsprecher
- 12: Multikopter
- 13: Rotor
- 14: Standfuß
- 15: Detektionsbereich
- 16: Markierungs-Vorrichtung
- 17: Sende/Empfangseinheit
- 18: Gondel (Ballon)
- 19: Akku
- 20: Temperatur-Sensor, T-Sensor, Pyrometer
- 21: Entfernungs-Sensor
- 22: Testfläche
- 23: GPS-Sensor
- 24: Kabel
- 25: Lichtstrahl-Quelle
- 26: Lichtstrahl
- 27: Injektions-Vorrichtung
- 28: Aufsteckeinheit
- 29: Ortungssensor
- 30: Auswerte-Einheit

- 40: Anzeige-Einheit, Display
- 41: Eingabe-Einheit

- 50: Tier-Identifikationsmittel

- 60: Zentralstation, Daten-Verarbeitungseinheit

- 70: Messstellen-Identifikationsmittel

- 80: Speichereinheit

- 90: Sende/Empfangseinheit

- 100: selbstfahrender Futter-Roboter

- F: Futter
- M: Mobiltelefon
- T: Tier

## Patentansprüche

1. **Vorrichtung** zur berührungslosen Ermittlung, also Messen, der Körpertemperatur eines freilaufenden Tieres (T), insbesondere Nutztieres in einem Gehege oder im Freiland und das nicht ständig bestimmte Futterstellen oder Melkstände aufsucht, wobei die Vorrichtung aufweist:
- eine Lokalisierung-Einheit, um zu vermessende Tiere auch in einem weitläufigen Areal wie Freiland, einer Weide oder einem Gehege zunächst ausfindig zu machen,
- ein Tieridentifikationsmittel (50),
- einen berührungslos arbeitenden Temperatursensor (20) zur Messung der Oberflächen-Temperatur an mindestens einer Messstelle des Körpers des Tieres (T),
- ein Sensor-Fahrzeug (1), welches den Temperatursensor (20) trägt, zur Annäherung an das Tier (T),
- eine Auswerteeinheit (30), die mit dem Temperatursensor (20) signaltechnisch in Verbindung steht, und die die vom Temperatursensor (20) gemessene Oberflächen-Temperatur erfasst, wobei die Auswerteeinheit (30) dazu eingerichtet ist, Temperaturdaten einer bestimmten Messung, einem bestimmten Tier, zuzuordnen und zu speichern.
- der Temperatursensor (20) mindestens zwei Sensorelemente (20a, b) umfasst, die vom Sensor-Fahrzeug (1) in unterschiedliche Richtungen ausgerichtet sind, so dass sie bei gleicher Position des Sensor-Fahrzeugs (1) die Oberflächen-Temperatur an unterschiedlichen Messstellen (6a, b) des Körpers des Tieres (T) oder aus unterschiedlichen Richtungen messen können,
- die mehreren Temperatur-Sensoren (20) einzeln hinsichtlich ihrer Meßrichtung ansteuerbar ausgebildet sind.

2. Vorrichtung nach Anspruch 1,
**dadurch gekennzeichnet, dass**
das Sensor-Fahrzeug
- eine Sende-/Empfangseinheit (40) aufweist, die mit dem Temperatursensor (20) verbunden ist und die mit der Auswerteeinheit (30), insbesondere drahtlos, signaltechnisch verbunden ist.

3. Vorrichtung nach einem der vorhergehenden Ansprüche,
**dadurch gekennzeichnet, dass**
das Sensor-Fahrzeug (1)
- ein Landfahrzeug,
insbesondere ein Rad-Fahrzeug (2), Raupen-Fahrzeug (2) oder Schreit-Fahrzeug (3) ist
- oder ein Luftfahrzeug ist,
insbesondere ein Hubschrauber, ein Multikopter (12), ein Ballon (7) oder ein Flugzeug ist.

4. Vorrichtung nach einem der vorhergehenden Ansprüche,
**dadurch gekennzeichnet, dass**
- der Temperatursensor (20) ein Pyrometer (20), insbesondere ein Infrarot-Pyrometer, und/oder eine Wärmebildkamera und/oder ein Infrarotsensor ist.

5. Vorrichtung nach einem der vorhergehenden Ansprüche,
**dadurch gekennzeichnet, dass**
- die Vorrichtung einen Ortungssensor (29) für Tiere (T) umfasst, der insbesondere
- entweder Bestandteil des Sensor-Fahrzeugs (1) ist
- oder Bestandteil eines Ortungs-Fahrzeugs ist.

6. Vorrichtung nach einem der vorhergehenden Ansprüche,
**dadurch gekennzeichnet, dass**
die Vorrichtung
- eine Eingabeeinheit zur Eingabe einer Tieridentität durch einen Nutzer und/oder
- die Auswerteeinheit (30) als die Tieridentifikationsmittel (50) eingerichtet ist.

7. Vorrichtung nach Anspruch 6,
**dadurch gekennzeichnet, dass**
- das Tieridentifikationsmittel (50) auf Entfernung funktionsfähig ist, und/oder
- das Tieridentifikationsmittel (50) einen Scanner, einen Barcode-Scanner, einen RFID-Scanner (50), einen Ohrmarken-Scanner und/oder eine Kamera (10) umfasst.
und/oder
- das Tieridentifikationsmittel (50) eingerichtet ist zur Gesichtserkennung.

8. Vorrichtung nach einem der vorhergehenden Ansprüche,
**dadurch gekennzeichnet, dass**
- der Temperatursensor (20) als Aufsteckeinheit (28) und/oder Teil einer Aufsteckeinheit (28) für ein Mobiltelefon (M), insbesondere ein Smartphone, ausgelegt ist,
- wobei das Mobiltelefon (M) besonders bevorzugt eingerichtet ist als Auswerteeinheit (30) und/oder Anzeigeeinheit (40).

9. Vorrichtung nach einem der vorhergehenden Ansprüche,
**dadurch gekennzeichnet, dass**
die Auswerte-Einheit (30) eingerichtet ist, auf Grund eines Anstiegs einer gemessenen Umgebungs-Temperatur oder eines Ansprechens eines mit dem Temperatursensor (20) verbundenen oder darin integrierten Bewegungssensors und/oder Mikrophons, eine Messung der Oberflächen-Temperatur durchzuführen und/oder eine gemessene Oberflächen-Temperatur an die Auswerteeinheit (30) zu senden.

10. Vorrichtung nach einem der vorhergehenden Ansprüche,
**dadurch gekennzeichnet, dass**
die Auswerteeinheit (30) dazu eingerichtet ist, basierend auf der Oberflächen-Temperatur eines bestimmten Tieres (6) Folgemaßnahmen auszulösen, wie beispielsweise die Abgabe von Warnsignalen, insbesondere
- optischen und/oder akustischen und/oder elektronischen Warnsignalen, und/oder Änderung eines Datenbankeintrags des bestimmten Tieres, und/oder
- Abgabe von Benachrichtigungen, insbesondere elektronischen Benachrichtigungen,
und/oder
Verabreichung von besonderen Tierpflegemitteln oder Medikamenten, insbesondere in Form von Injektionen oder als Futter.

11. Vorrichtung nach einem der vorhergehenden Ansprüche, **gekennzeichnet durch**
eine Datenverarbeitungseinheit (60) zur Speicherung und Pflege eines Tierdatenbestandes, bevorzugt in einer Zentralstation (60),
- wobei die Sende- und/oder Anzeigeeinheit (90) insbesondere eingerichtet ist, Temperaturdaten mit Referenz auf das zugehörige bestimmte Tier (T) an die Datenverarbeitungseinheit (60) zu senden,
und/oder
- wobei die Datenverarbeitungseinheit (60) insbesondere dazu eingerichtet ist, Befehlssignale an Auswerteeinheit (30) und/oder die Sende- und Anzeigeeinheit (40) zu senden, um anhand des Tierdatenbestandes als erforderlich ermittelte Vorgänge auszulösen.

12. Vorrichtung nach einem der vorhergehenden Ansprüche,
**dadurch gekennzeichnet, dass**
die Auswerteeinheit (30) dazu eingerichtet ist, in Abwesenheit eines Tieres (T) Messungen von Neutraltemperaturen, insbesondere an einer Testfläche (22,) auszuführen, und bei ungewöhnlichen Neutraltemperaturen, insbesondere in Anbetracht einer Raumtemperatur, welche beispielsweise an die Auswerteeinheit (30) regelmäßig von einem Raumtemperatursensor übermittelt wird, ein Störungssignal auszulösen und abzugeben, insbesondere an die Sende- und Anzeigeeinheit (40).

13. Vorrichtung nach einem der vorhergehenden Ansprüche,
**dadurch gekennzeichnet, dass**
- die Vorrichtung dazu eingerichtet ist, die Entfernung zwischen Temperatursensor (20) und dem Tier (T), insbesondere der mindestens einen Messstelle des Körpers des Tieres (T), zu erfassen bzw. zu ermitteln,
- wobei bevorzugt die Auswerteeinheit (30) und/oder die Datenverarbeitungseinheit (60) ferner dafür ausgelegt ist, die Entfernung zu speichern, und/oder eine korrigierte Temperatur unter Verwendung von wenigstens der gemessenen Oberflächen-Temperatur und der Entfernung zu bestimmen.

14. **Verfahren** zum Ermitteln der Körpertemperatur eines freilaufenden sich frei bewegenden Tieres (T), dass sich in einem Gehege oder im Freiland auffällt und nicht ständig bestimmte Futterstellen oder Melkstände aufsucht, bei Nutzung einer nach einem der Ansprüche 1 bis 13 ausgebildeten Vorrichtung, umfassend die Schritte:
- Lokalisieren des Tieres (T),
- Identifizieren des Tieres (T),
- Heranbewegen des Sensor-Fahrzeuges (1) an das Tier (T) bis auf eine solche Entfernung, dass das Messen der Oberflächen-Temperatur des Tieres (T) mit dem berührungslosen Temperatur-Sensor (20) möglich ist,
- berührungsloses Messen der Oberflächen-Temperatur des Tieres (T) mittels mehreren Temperatur-Sensoren (20) der Vorrichtung,
- Speicherung und Vergleich der gemessenen Oberflächen-Temperatur mit früher gemessenen Oberflächen-Temperaturen desselben Tieres (T).
- die mehreren Temperatur-Sensoren (20) einzeln hinsichtlich ihrer Meßrichtung angesteuert werden.

15. Verfahren nach Anspruch 14,
**dadurch gekennzeichnet, dass**
- . Übersendung des Messergebnisses an eine Auswerteeinheit (60) und/oder
- der Vergleich der gemessenen Oberflächen-Temperatur mit früher gemessenen Oberflächen-Temperaturen desselben Tieres an der gleichen Stelle des Körpers des Tieres (T) durchgeführt wird,
- die Auslösung einer Folgefunktion abhängig von dem Messergebnis oder dem Ergebnis des Vergleichs durchgeführt wird,
und/oder
- eine Speicherung der gemessenen Oberflächen-Temperatur des Tieres (T), bevorzugt in Verbindung mit anderen Daten wie etwa Ort, Uhrzeit, Datum, ggfs. Fütterungsmenge, ggfs. Verweildauer des Tieres in einer Tierversorgungseinrichtung und/oder Tierpflegeeinrichtung, ggfs. Menge der gemolkenen Milch;
- wobei alle Schritte mindestens einmal pro Woche, bevorzugt mindestens einmal pro 24 Stunden, ausgeführt werden.

16. Verfahren nach Anspruch 14 oder 15,
**dadurch gekennzeichnet, dass**
der Temperatur-Sensor (20) durch Messen der, insbesondere bekannten, Oberflächen-Temperatur einer Testfläche (22) kalibriert wird.

## Claims

1. Device for the contactlessly determining, i.e. measuring, the body temperature of a free-roaming animal (T), in particular a farm animal (T), in an enclosure or in open land and that does not constantly visit specific feeding places or milking parlours, wherein the device comprises:
- a locating unit to initially locate animals to be measured even in a large area such as open land, a pasture or an enclosure,
- an animal identification means (50),
- a non-contact temperature sensor (20) for measuring the surface temperature at at least one measuring point on the body of the animal (T),
- a sensor vehicle (1), which supports the temperature sensor (20), for approaching the animal (T),
- an evaluation unit (30) which is connected to the temperature sensor (20) by means of signal technology and detects the surface temperature measured by the temperature sensor (20), wherein the evaluation unit (30) is designed to assign and store temperature data from a specific measurement to a specific animal,
- the temperature sensor (20) comprises at least two sensor elements (20a, b) which are oriented in different directions from the sensor vehicle (1) so that they can measure the surface temperature at different measuring points (6a, b) on the body of the animal (T) or from different directions when the sensor vehicle (1) is in the same position,
- the plurality of temperature sensors (20) are designed to be individually controllable with regard to their measuring direction.

2. Device according to claim 1,
**characterised in that**
the sensor vehicle
- comprises a transmitting/receiving unit (40) which is connected to the temperature sensor (20) and is connected to the evaluation unit (30), in particular wirelessly, by means of signal technology.

3. Device according to either of the preceding claims,
**characterised in that**
the sensor vehicle (1)
- is a land vehicle,
in particular is a wheeled vehicle (2), tracked vehicle (2) or walking vehicle (3),
- or an aircraft,
in particular is a helicopter, a multicopter (12), a balloon (7) or an aeroplane.

4. Device according to any of the preceding claims,
**characterised in that**
- the temperature sensor (20) is a pyrometer (20), in particular an infrared pyrometer, and/or a thermal imaging camera and/or an infrared sensor.

5. Device according to any of the preceding claims,
**characterised in that**
- the device comprises a location sensor (29) for animals (T), which in particular
- is either part of the sensor vehicle (1)
- or is part of a locating vehicle.

6. Device according to any of the preceding claims,
**characterised in that**
the device
- is an input unit for inputting an animal identity by means of a user
and/or
- the evaluation unit (30) is designed as the animal identification means (50).

7. Device according to claim 6,
**characterised in that**
- the animal identification means (50) is functional at a distance,
and/or
- the animal identification means (50) comprises a scanner, a barcode scanner, an RFID scanner (50), an ear tag scanner and/or a camera (10),
and/or
- the animal identification means (50) is designed for facial recognition.

8. Device according to any of the preceding claims,
**characterised in that**
- the temperature sensor (20) is designed as a plug-in unit (28) and/or part of a plug-in unit (28) for a mobile phone (M), in particular a smartphone,
- the mobile phone (M) particularly preferably being designed as an evaluation unit (30) and/or display unit (40).

9. Device according to any of the preceding claims,
**characterised in that**
the evaluation unit (30) is designed to carry out a measurement of the surface temperature and/or to transmit a measured surface temperature to the evaluation unit (30) due to an increase in a measured ambient temperature or a response of a motion sensor and/or microphone connected to or integrated in the temperature sensor (20).

10. Device according to any of the preceding claims,
**characterised in that**
the evaluation unit (30) is designed to trigger follow-up measures on the basis of the surface temperature of a particular animal (6), such as outputting warning signals, in particular
- optical and/or acoustic and/or electronic warning signals, and/or changing a database entry for the specific animal,
and/or
- outputting notifications, in particular electronic notifications,
and/or
administering special animal care products or medications, in particular in the form of injections or as feed.

11. Device according to any of the preceding claims,
**characterised by**
a data processing unit (60) for storing and maintaining an animal dataset, preferably in a central station (60),
- the transmitting and/or display unit (90) in particular being designed to transmit temperature data with reference to the associated particular animal (T) to the data processing unit (60),
and/or
- the data processing unit (60) in particular being designed to transmit command signals to the evaluation unit (30) and/or the transmitting and display unit (40) in order to trigger processes which are determined to be necessary on the basis of the animal dataset.

12. Device according to any of the preceding claims,
**characterised in that**
the evaluation unit (30) is designed to carry out measurements of neutral temperatures, in particular on a test surface (22), in the absence of an animal (T), and to trigger and output a fault signal, in particular to the transmitting and display unit (40), in the event of unusual neutral temperatures, in particular taking into consideration a room temperature which, for example, is regularly sent to the evaluation unit (30) by a room temperature sensor.

13. Device according to any of the preceding claims,
**characterised in that**
- the device is designed to detect or determine the distance between the temperature sensor (20) and the animal (T), in particular the at least one measuring point on the body of the animal (T),
- the evaluation unit (30) and/or the data processing unit (60) preferably being further designed to store the distance and/or to determine a corrected temperature using at least the measured surface temperature and the distance.

14. Method for determining the body temperature of a free-roaming, freely moving animal (T), which is out in an enclosure or in open land and does not constantly visit specific feeding places or milking parlours, using a device designed according to any of claims 1 to 13, comprising the steps of:
- locating the animal (T),
- identifying the animal (T),
- moving the sensor vehicle (1) towards the animal (T) to such a distance that the surface temperature of the animal (T) can be measured using the contactless temperature sensor (20),
- contactlessly measuring the surface temperature of the animal (T) by means of a plurality of temperature sensors (20) of the device,
- storing and comparing the measured surface temperature with previously measured surface temperatures of the same animal (T),
- the plurality of temperature sensors (20) are individually controlled with regard to their measuring direction.

15. Method according to claim 14,
**characterised in that**
- sending the measurement result to an evaluation unit (60)
and/or
- comparing the measured surface temperature with previously measured surface temperatures of the same animal at the same point on the body of the animal (T) is carried out,
- triggering a subsequent function depending on the measurement result or the result of the comparison is carried out,
and/or
- storing the measured surface temperature of the animal (T), preferably in conjunction with other data, such as location, time, date, if applicable amount of feeding, if applicable length of stay of the animal in an animal treatment facility and/or animal care facility, or if applicable amount of milk milked;
- all steps being carried out at least once a week, preferably at least once every 24 hours.

16. Method according to either claim 14 or claim 15,
**characterised in that**
the temperature sensor (20) is calibrated by measuring the, in particular known, surface temperature of a test surface (22).

## Revendications

1. Dispositif de détermination sans contact, donc de mesure, de la température corporelle d'un animal (T) en stabulation libre, en particulier d'un animal de rente (T) dans un enclos ou en pleine nature, qui ne se rend pas en permanence à des places d'affouragement ou dans des salles de traite, dans lequel le dispositif présente :
- une unité de localisation pour d'abord localiser des animaux à jauger également dans un vaste domaine, comme la pleine nature, dans un pâturage ou dans un enclos,
- un moyen d'identification d'animal (50),
- un capteur de température (20) fonctionnant sans contact destiné à mesurer la température de surface sur au moins un point de mesure du corps de l'animal (T),
- un véhicule à capteur (1), lequel supporte le capteur de température (20), destiné à approcher l'animal (T),
- une unité d'évaluation (30), qui est reliée par une technique de signalisation au capteur de température (20) et qui détecte la température de surface mesurée par le capteur de température (20), dans lequel l'unité d'évaluation (30) est mise au point pour associer des données de température à une mesure donnée, à un animal donné et pour les stocker,
- le capteur de température (20) comprend au moins deux éléments de capteur (20a, b), qui sont orientés par le véhicule à capteur (1) dans différentes directions de telle sorte qu'ils peuvent mesurer dans une position identique du véhicule à capteur (1) la température de surface sur différents points de mesure (6a, b) du corps de l'animal (T) ou depuis différentes directions,
- les plusieurs capteurs de température (20) sont réalisés de manière à pouvoir être pilotés individuellement quant à leur direction de mesure.

2. Dispositif selon la revendication 1,
**caractérisé en ce que**
le véhicule à capteur
- présente une unité d'envoi/de réception (40), qui est reliée au capteur de température (20) et qui est reliée par une technique de signalisation, en particulier sans fil, à l'unité d'évaluation (30).

3. Dispositif selon l'une quelconque des revendications précédentes,
**caractérisé en ce que**
le véhicule à capteur (1)
- est un véhicule terrestre,
en particulier un véhicule à roues (2), un véhicule à chenilles (2) ou un véhicule ambulant (3),
- ou est un aéronef,
en particulier un hélicoptère, un multicoptère (12), une montgolfière (7) ou un avion.

4. Dispositif selon l'une quelconque des revendications précédentes,
**caractérisé en ce que**
- le capteur de température (20) est un pyromètre (20), en particulier un pyromètre infrarouge, et/ou une caméra thermique et/ou un capteur infrarouge.

5. Dispositif selon l'une quelconque des revendications précédentes,
**caractérisé en ce que**
- le dispositif comprend un capteur de localisation (29) d'animaux (T), qui en particulier
- soit fait partie intégrante du véhicule à capteur (1),
- soit fait partie intégrante d'un véhicule de localisation.

6. Dispositif selon l'une quelconque des revendications précédentes,
**caractérisé en ce que**
le dispositif
- est une unité de saisie destinée à la saisie d'une identité d'un animal par un utilisateur et/ou
- est une unité d'évaluation (30) mise au point en tant que moyen d'identification d'animal (50).

7. Dispositif selon la revendication 6,
**caractérisé en ce que**
- le moyen d'identification d'animal (50) peut fonctionner à distance,
et/ou
- le moyen d'identification d'animal (50) comprend un scanner, un scanner à code à barres, un scanner RFID (50), un scanner d'étiquette auriculaire et/ou une caméra (10), et/ou
- le moyen d'identification d'animal (50) est mis au point pour la reconnaissance faciale.

8. Dispositif selon l'une quelconque des revendications précédentes,
**caractérisé en ce que**
- le capteur de température (20) est conçu en tant qu'une unité enfichable (28) et/ou en tant que partie d'une unité enfichable (28) pour un téléphone mobile (M), en particulier un téléphone intelligent,
- dans lequel le téléphone mobile (M) est mis au point de manière particulièrement préférée en tant qu'unité d'évaluation (30) et/ou en tant qu'unité d'affichage (40).

9. Dispositif selon l'une quelconque des revendications précédentes,
**caractérisé en ce que**
l'unité d'évaluation (30) est mise au point pour effectuer une mesure de la température de surface en raison d'une hausse d'une température ambiante mesurée ou en réponse à un capteur de mouvement et/ou à un microphone relié au capteur de température (20) ou intégré dans celui-ci et/ou pour envoyer une température de surface mesurée à l'unité d'évaluation (30).

10. Dispositif selon l'une quelconque des revendications précédentes,
**caractérisé en ce que**
l'unité d'évaluation (30) est mise au point pour déclencher, sur la base de la température de surface d'un animal (6) donné, des mesures de suivi, comme l'émission de signaux d'avertissement, en particulier
- de signaux d'avertissement optiques et/ou acoustiques et/ou électroniques et/ou une modification d'un enregistrement dans la base de données de l'animal donné,
et/ou
- l'émission de messages, en particulier de messages électroniques,
et/ou
l'administration de soins pour animaux ou de médicaments spécifiques, en particulier sous la forme d'injections ou en tant qu'aliments.

11. Dispositif selon l'une quelconque des revendications précédentes,
**caractérisé par**
une unité de traitement de données (60) destinée à stocker et à maintenir une base de données d'animaux, de manière préférée dans un poste central (60),
- dans lequel l'unité d'envoi et/ou d'affichage (90) est mise au point en particulier pour envoyer des données de température avec référence à l'animal (T) donné associé à l'unité de traitement de données (60),
et/ou
- dans lequel l'unité de traitement de données (60) est mise au point en particulier pour envoyer des signaux de commande à l'unité d'évaluation (30) et/ou à l'unité d'envoi et d'affichage (40) pour déclencher des opérations déterminées comme étant nécessaires à l'aide de la base de données d'animaux.

12. Dispositif selon l'une quelconque des revendications précédentes,
**caractérisé en ce que**
l'unité d'évaluation (30) est mise au point pour exécuter en l'absence d'un animal (T) des mesures de températures neutres, en particulier sur une zone de test (22), et pour déclencher, en présence de températures neutres inhabituelles, en particulier au regard d'une température ambiante, laquelle est transférée régulièrement par un capteur de température ambiante par exemple à l'unité d'évaluation (30), un signal de défaut et l'envoyer en particulier à l'unité d'envoi et d'affichage (40).

13. Dispositif selon l'une quelconque des revendications précédentes,
**caractérisé en ce que**
- le dispositif est mis au point pour détecter ou pour déterminer l'éloignement entre le capteur de température (20) et l'animal (T), en particulier l'au moins un point de mesure du corps de l'animal (T),
- dans lequel de manière préférée l'unité d'évaluation (30) et/ou l'unité de traitement de données (60) est conçue en outre pour stocker un éloignement, et/ou pour définir une température corrigée en utilisant au moins la température de surface mesurée et l'éloignement.

14. Procédé pour déterminer la température corporelle d'un animal (T) en stabulation libre se déplaçant librement, qui se trouve dans un enclos ou en pleine nature et ne se rend pas en permanence à des emplacements d'affouragement ou des salles de traite donnés, en utilisant un dispositif réalisé selon l'une quelconque des revendications 1 à 13, comprenant les étapes :
- de localisation de l'animal (T),
- d'identification de l'animal (T),
- de rapprochement du véhicule à capteur (1) de l'animal (T) jusqu'à un tel éloignement que la mesure de la température de surface de l'animal (T) soit possible avec le capteur de température (20) sans contact,
- de mesure sans contact de la température de surface de l'animal (T) au moyen de plusieurs capteurs de température (20) du dispositif,
- de stockage et de comparaison de la température de surface mesurée à des températures de surface, mesurées antérieurement, du même animal (T),
dans lequel
- les plusieurs capteurs de température (20) sont pilotés individuellement quant à leur direction de mesure.

15. Procédé selon la revendication 14,
**caractérisé en ce que**
- le résultat de mesure est transmis à une unité d'évaluation (60) et/ou
- la comparaison de la température de surface mesurée à des températures de surface mesurées antérieurement du même animal est effectuée sur le même point du corps de l'animal (T),
- le déclenchement d'une fonction de suivi est effectué en fonction du résultat de mesure ou du résultat de la comparaison,
et/ou
- la température de surface mesurée de l'animal (T), de manière préférée en lien avec d'autres données comme le lieu, l'heure, la date, éventuellement la quantité d'aliments, éventuellement la durée de séjour de l'animal dans une installation d'alimentation de l'animal et/ou une installation de soin de l'animal, éventuellement la quantité du lait trait, est stockée ;
- dans lequel toutes les étapes sont exécutées au moins une fois par semaine, de manière préférée au moins une fois toutes les 24 heures.

16. Procédé selon la revendication 14 ou 15,
**caractérisé en ce que** le capteur de température (20) est étalonné en mesurant la température de surface, en particulier connue, d'une surface de test (22).
